# EUROPEAN PATENT APPLICATION

(11) **EP 3 097 893 A1**
(43) Date of publication of application: **30.11.2016**
(21) Application number: 15382278.8
(22) Date of filing: 26.05.2015
(51) Int. Cl.: A61F 9/007

(54) **MEDICAL DEVICE FOR TREATING OBSTRUCTION OF THE LACRIMAL DUCTS**

(71) Applicant: Fundacion Tekniker, 20600 Eibar (Guipuzkoa) (ES); Palomino Munoz, Antonio, 28009 Madrid (ES)
(72) Inventor: PALOMINO MÚÑOZ, Antonio, 28009 Madrid (ES); OÑATE GUTIERREZ, Roberto, 20600 Eibar, Guipúzcoa (ES)
(74) Representative: Balder IP Law, S.L.

(57) **Abstract**

A device for treating obstruction of the lacrimal ducts, comprising a cord (50, 80) made of biocompatible material, the cord (50) having a first end (501, 801), and a second end (502, 802) opposite the first one, said first end (501, 801) being blunt or rounded. The cord (50, 80) is designed to be inserted first end (501) first through an eye's lacrimal punctum (12, 15) along the respective canaliculus (13, 16) until reaching the nasal cavity. The cord (50, 80) comprises a plurality of housings (51, 52, 53; 81, 82, 83) ending in respective cul-de-sacs, designed to receive an end of a rod (60) such that, when an end of the rod (60) is inserted into a housing (51, 52, 53; 81, 82, 83) and said end of the rod is pressed into the housing, the cord (50, 80) is pushed by the rod (60) from the outer part of the cord (50, 80) into the lacrimal duct.

## Description

### APPLICATION FIELD

The present invention falls within the field of medical devices, and in particular surgical devices for treating obstruction of the lacrimal ducts.

### BACKGROUND OF THE INVENTION

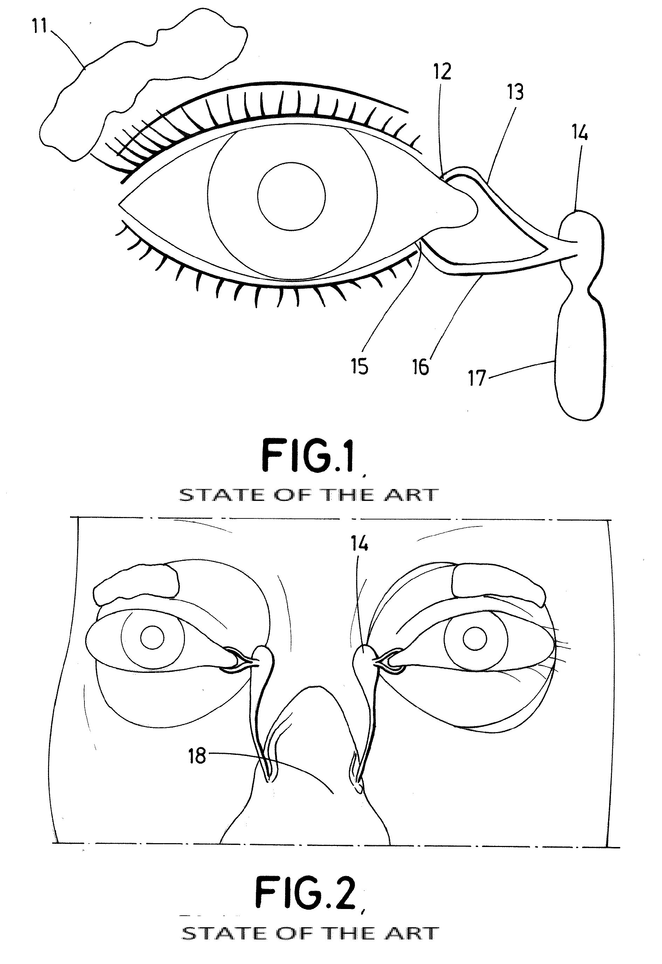

Figure 1 shows a diagram of the human eye. Tears are produced mainly in the lacrimal gland 11, situated in the upper, outer portion inside the orbit. Once they have fulfilled their protective function, i.e. moistening and lubricating the eyeball, they drain into the nasal cavity through what is known as the lacrimal ducts. The lacrimal ducts begin at two puncta lacrimalia (the upper lacrimal punctum or point 12 and the lower lacrimal punctum or point 15), and continue, respectively, through the upper 13 (in the upper eyelid) and lower 16 (in the lower eyelid) lacrimal canaliculi or canals, which are small conduits that channel or drain the tears into the lacrimal sac 14.These two canaliculi or canals 13 16 converge into a single canal before opening into the lacrimal sac 14, a receptacle that carries out a very important role in the correct functioning of tear drainage. The lacrimal sac 14 connects the lacrimal canals 13 16 to the nasolacrimal duct (sometimes lacrimonasal duct) 17, which conducts this fluid toward the nasal cavity 18 (illustrated in figure 2). In other words, the nasolacrimal duct 17 empties into the nasal cavity at the height of the inferior nasal turbinate. The lacrimal sac 14 basically begins to function when there is a large quantity of tears, which the lacrimal sac 14 pumps in and out, guided by the orbicularis palpebrarum muscle, during blinking. Whereas the upper portion of the lacrimal sac 14 is rounded and closed off, the lower portion continues into the nasolacrimal duct 17. Figure 2 shows the diagram in figure 1, where one may also observe how the nasolacrimal duct 17 empties into the nasal cavity 18.

Epiphora, commonly known as watering eyes, has many causes. One of the most frequent is obstruction of the lacrimal ducts. Children may be born with an impermeable (i.e. obstructed) lacrimal duct. In adults there may be stenosis (narrowness) of the lacrimal duct, or total obstruction. Obstruction of the lacrimal duct in children and recent stenosis or obstruction in adults may be treated with bicanalicular silicone intubation of the entire lacrimal duct down to the inferior nasal turbinate.

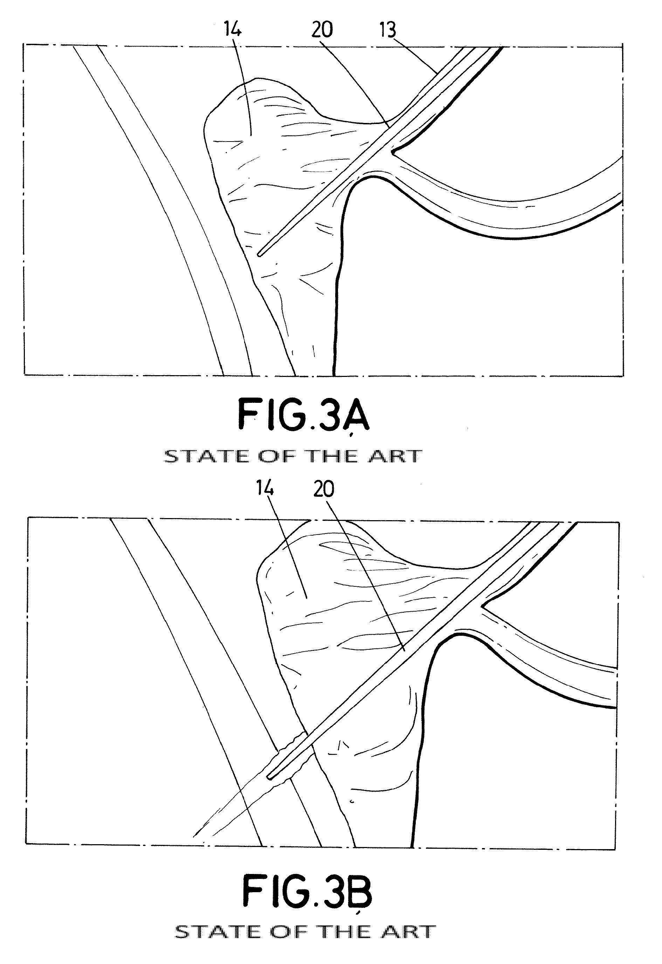

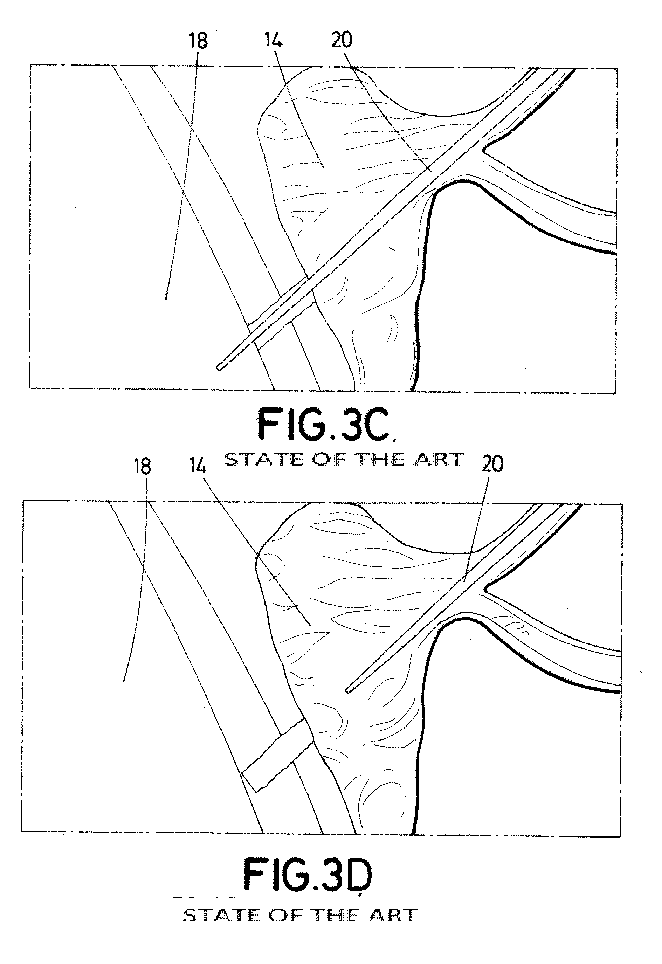

When this treatment does not cure the condition, a surgical operation is performed called dacryocystorhinostomy, which consists of circumventing the obstruction - usually at the height of the nasolacrimal duct - by making a perforation that connects the lacrimal sac to the nasal cavity at the height of the middle nasal turbinate, thus creating a new canal. The lacrimal sac can be connected to the nasal cavity in a variety of ways:
1) Through traditional surgery, which consists of making an incision in the skin, perforating the nasal bone, and stitching the sac to the nasal mucosa (traditional or external dacryocystorhinostomy).
2) Through endonasal surgery, which is similar to the traditional surgery but is carried out through the nose, without making an incision in the skin (endonasal dacryocysto rh i n osto my).
3) Through laser surgery (transcanalicular laser dacryocystorhinostomy), described schematically in figures 3A-3D. It does not leave any scarring on the skin. It is carried out using a laser probe measuring approximately 600 microns, which is inserted through the lacrimal punctum, reaching the lacrimal sac by moving along the lacrimal canal. The sac and bone are perforated, passing through them until reaching the nasal cavity.

Figures 3A-3D schematically show how this operation is carried out. One of the puncta lacrimalia is dilated (normally the upper punctum 12) with a lacrimal probe or punctum dilator. Next, a laser probe is inserted up to the lacrimal sac 14. The wall of the latter is perforated (figure 3B), for example with a laser probe, along with the nasal wall, in order to reach the nasal cavity 18 (figure 3C).

The advantage of this technique is that it is less aggressive - for example, there is hardly any bleeding - but its disadvantage is that the diameter of the opening made in order to reach the nose is small, due to the calibre of the laser fibre. For this reason, a stent is temporarily put in place, for example one made of silicone (ophthalmologists need the help of an otorhinolaryngologist to pull out the two tips of the stent). In other words, to keep the new duct from closing up, it is advisable to introduce a tube made of biocompatible material, which will stay inside the canal for approximately six months, so that it does not close up. This silicone stent may be bicanalicular (passing through both canaliculi) or monocanalicular (passing through just one canaliculus). In the latter case, the lower canaliculus is preferably be used.

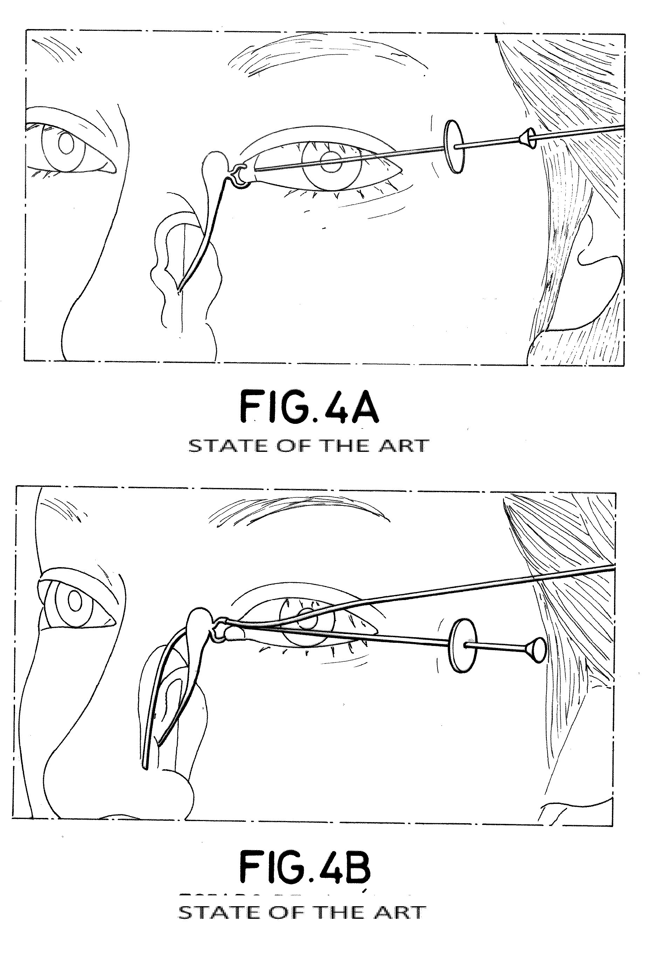

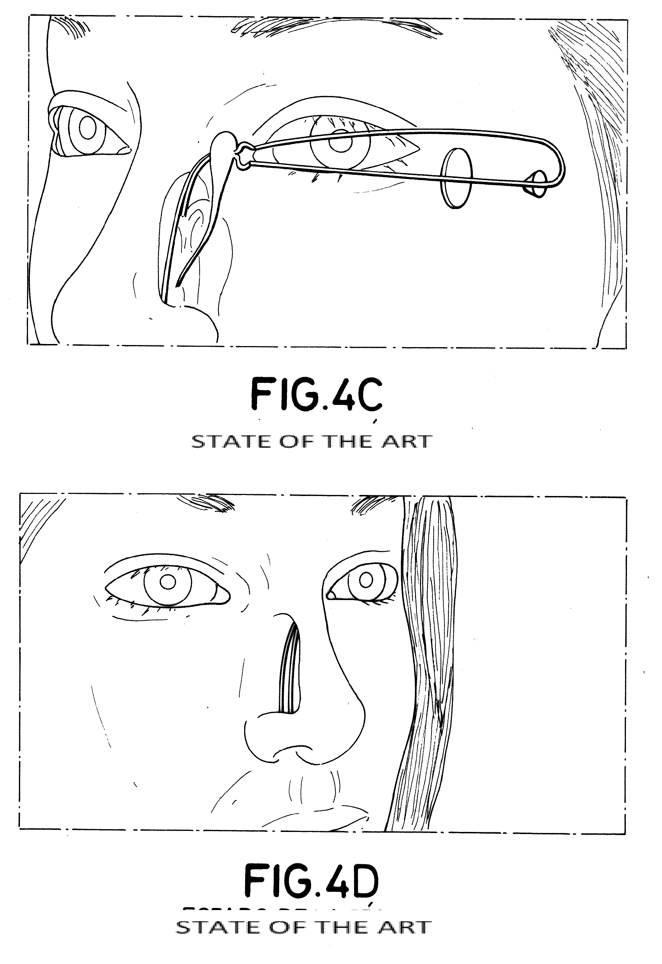

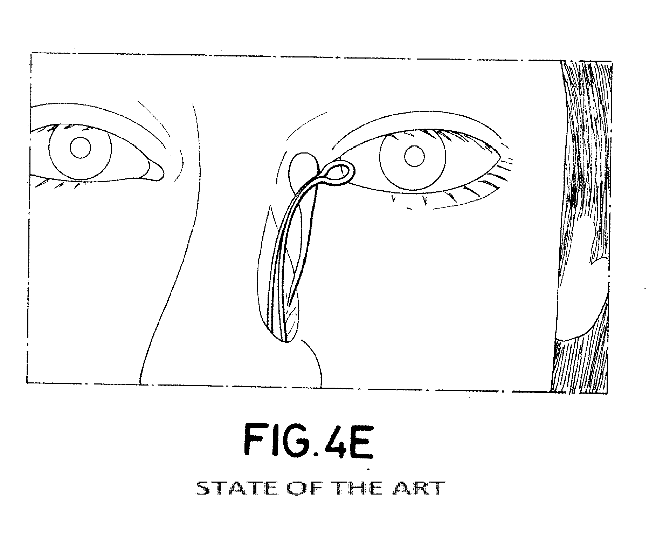

In conventional bicanalicular stents, the two ends of the stent are extracted through the nasal cavity and are then tied off. When the intubation or stent is monocanalicular, one end of the stent must have a plug to fit and anchor to the lacrimal punctum so that it does not fall out. Figures 4A-4E show how the first end of the tube is inserted along the upper canaliculus 13 (figures 4A-4B) whilst a second end is inserted along the lower canaliculus 16 (figure 4C). To do this, the tube is inserted, its ends typically being fitted to a mandrel or metal probe in order to pass through the lacrimal duct. Figures 4D and 4E show the bicanalicular version, where one may observe the tube hanging into the nasal cavity. The two ends that hang into the nose are tied off so that they will not come out. After several months inside the new duct, this tube may be extracted by cutting it in the nose and pulling on one of the ends. Alternatively, it may also be extracted by cutting it and pulling on one of the ends from the canaliculus.

The problem with this method is that it is difficult and rather aggressive to push the metal end of the silicone tube along the newly created (or natural) canal or duct until it protrudes into or may be grasped via the nostrils. This problem is most common in the bicanalicular version. To do so, forceps or other elements are inserted, through the nostrils, as far as possible into the nasal end of the newly created duct. This tends to give rise to injury to the nasal cavity or to the newly created lacrimal canal itself.

The utility model application published as ES1027204-U describes a stent for treating obstruction of the natural lacrimal duct. This stent is inserted into the lacrimal punctum and opens the lacrimal duct up to the inside of the nose. The stent is made up of a cylindrical body and also has a silicone anchoring button that fits to the lacrimal canal, so that the secretions run through the stent's inner duct up to the nose. The cylindrical body allows a metal probe to pass through it up to the nose. Likewise, European patent EP2086479-B1 describes a monocanaliculonasal intubation assembly to unclog a blocked lacrimal canal, made up of a stiff mandrel and a hollow tube that is less stiff than the mandrel, intended to be inserted inside the hollow tube. The assembly also includes a stop plug. There is a hole in the side of the hollow tube for the mandrel to pass through. There are some drawbacks to this monocanalicular technique, such as the following. On the one hand, it is necessary to manufacture devices with different sizes and previously use a calibrated metal probe to measure the length of the lacrimal duct being treated, so that the inserted stent is not too short (causing the treatment to fail) or too long, which would make it impossible to place the button in the lacrimal punctum, since the device would run up against the floor of the nasal cavity. Moreover, the plug for securing the device to the lacrimal punctum is not hollow and does not have the same inner diameter as the tube. This in turn causes the eyes to water for as long as the device remains in the lacrimal duct (several months), i.e. prolonging even further the problem it sets out to treat.

### DESCRIPTION OF THE INVENTION

The present invention seeks to solve the above-mentioned drawbacks by means of a device to combat lacrimal obstruction based on a stent that is an alternative to the known types.

A first aspect of the invention provides for a device for treating obstruction of the lacrimal ducts, comprising a cord made of biocompatible material, the cord having a first end, and a second end opposite the first one. The first end is blunt or rounded. The cord is designed to be inserted first end first through an eye's lacrimal punctum along the respective canaliculus until reaching the nasal cavity. The cord comprises a plurality of housings ending in respective cul-de-sacs, designed to receive the end of a rod such that, when an end of the rod is inserted into a housing and said end of the rod is pressed into the housing, the cord is pushed by the rod from the outer part of the cord into the lacrimal duct.

The housings are preferably aligned with one another, i.e. they are arranged along a common longitudinal axis of the cord.

The width or diameter of each housing preferably varies between 0.3 and 0.8 mm, and its length or depth varies between 3 and 10 mm.

The cord is preferably made out of silicon.

The cord preferably has a PVC coating.

In one possible embodiment, the device comprises a plug (or cap) designed to plug the lacrimal punctum through which the cord has been inserted, after the latter has been inserted into the lacrimal duct. The plug (or cap) is preferably joined to the second end of the cord.

In another possible embodiment, the second end of the cord is designed to be inserted through an eye's second lacrimal punctum along the respective canaliculus until reaching the nasal cavity. The length of cord that follows this second end comprises a plurality of housings ending in respective cul-de-sacs, designed to receive the end of a rod such that, when the end of the rod is pressed into the housing, the length of cord is pushed into the lacrimal duct. Preferably, in the vicinity of at least one of the housings of the cord, said housing further comprises at least one anchor in the form of a projection to help fix the cord in the lacrimal ducts once it has been inserted therein.

The cord is designed to be inserted along a natural lacrimal canal or duct or a lacrimal canal or duct made through surgery.

Another aspect of the invention provides a monocanalicular or bicanalicular intubation assembly, which comprises: a device such as the one described above; and a substantially stiff metal or Teflon® rod, comprising a blunt or rounded end designed to be inserted into one of the housings of the cord in order to move the cord forward along the lacrimal duct.

The diameter of the rod preferably varies between 0.3 and 0.8 mm.

Another aspect of the invention provides the use of the above-mentioned device or assembly in a surgical treatment.

A final aspect of the invention provides the above-mentioned device or assembly for use thereof in a surgical method that comprises creating an artificial lacrimal canal or using a natural canal between a patient's lacrimal sac and nasal cavity.

The advantages of the invention will become clearer with the aid of the description presented below.

### BRIEF DESCRIPTION OF THE DRAWINGS

As a complement to the description, and for the purpose of helping to make the characteristics of the invention more readily understandable, in accordance with a practical embodiment thereof, said description is accompanied by a set of figures constituting an integral part thereof, which by way of illustration and not limitation represent the following:
Figures 1 and 2 show a diagram of the human eye.
Figures 3A-3D show the steps conventionally followed to create a new nasolacrimal duct when there is an obstruction of the lacrimal ducts.
Figures 4A-4E show the typical way of inserting a double tube into the new duct, the creation of which is illustrated in figures 3A-3D.
Figure 5A shows a diagram of a medical device according to the present invention, in the monocanalicular version thereof, which has a cord, preferably made of silicone, a plug for anchoring to the lacrimal punctum, and a mandrel, preferably made of metal, so that it may be inserted through the lacrimal ducts.
Figure 5B shows the cord ending in an anchoring plug, of the medical device in figure 5A.
Figure 6A shows the plug to be fixed to the lacrimal punctum, of the device of figures 5A and 5B. The plug is continuous with the rest of the cord. This plug is preferably perforated so that the tears may pass through. The plug is joined such that it is substantially perpendicular to the longitudinal axis of the cord.
Figure 6B shows a plug to be fixed to the lacrimal punctum, of a medical device in accordance with another embodiment of the invention.
Figure 7 shows an assembly formed by cord and plug, in accordance with another possible embodiment of the invention.
Figure 8 shows the bicanalicular version of the invention, with its anchors and holes to insert the mandrel from the outside.
Figures 9A and 9B show a diagram of the anchors of the bicanalicular stent when it is in the lacrimal ducts, both natural and surgical.

### DESCRIPTION OF AN EMBODIMENT OF THE INVENTION

In this text, the word "comprises" and variants thereof (such as "comprising", etc.) should not be understood in an exclusive sense, i.e. they do not exclude the possibility of that which is described including other elements, steps, etc.

In the context of the present invention, the term "approximately" and related terms (such as "approximate," etc.) should be understood as indicative values that are very close to those that accompany said term. In other words, one should accept a deviation within the acceptable limits based on a precise value, as one skilled in the art will understand that said deviation based on the indicated values is inevitable due to imprecisions in measurement, etc. The same applies to the terms "around" and "substantially".

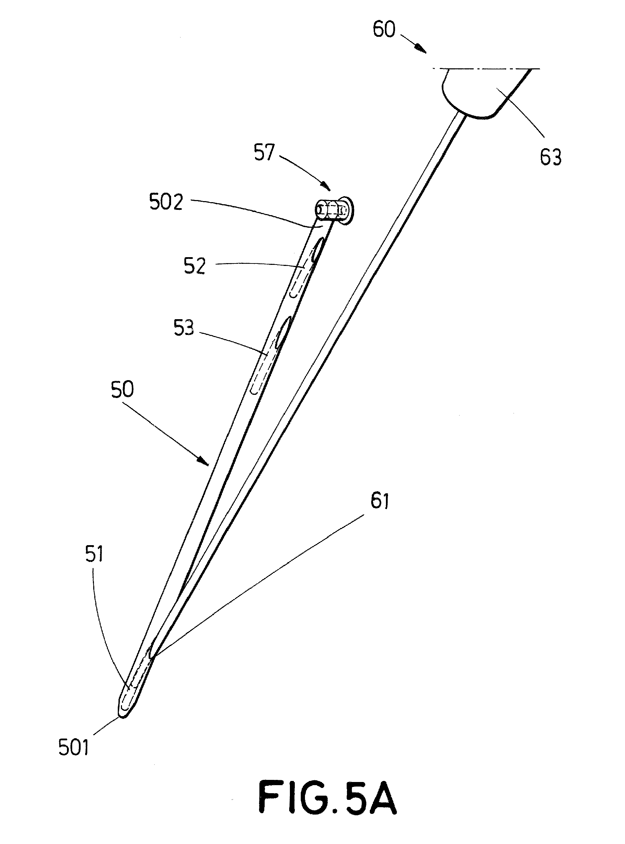

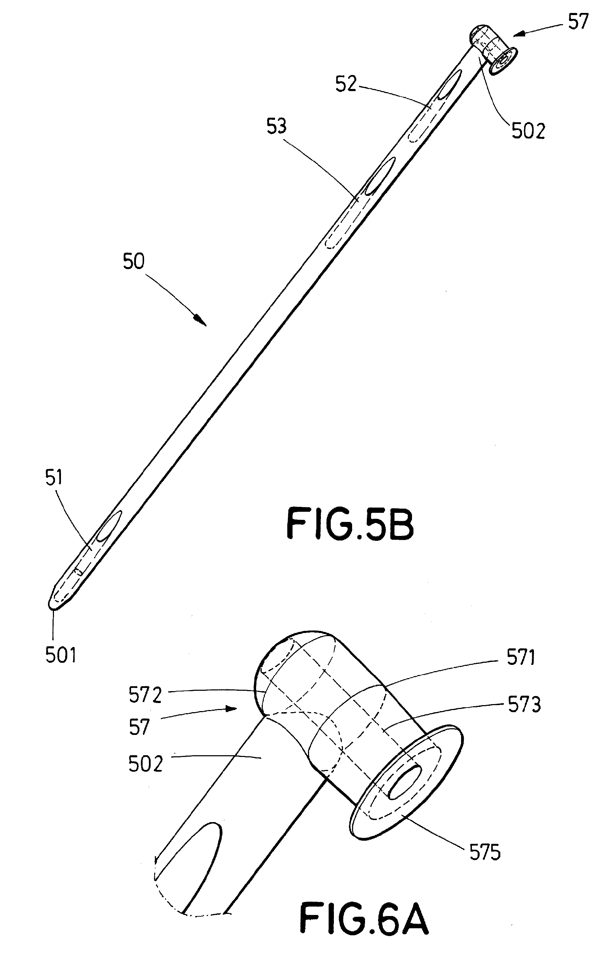

Figure 5A shows a diagram of a monocanalicular intubation assembly, in accordance with a possible embodiment of the invention. In figure 5A it may be seen how the assembly is formed by a solid (not hollow) cord, tube or stent 50 and a rod or mandrel 60. Figure 5B shows the cord 50 in figure 5A. The cord 50 is made of a biocompatible material. The cord is substantially flexible. The material it is made of is preferably a polymer, and more preferably silicone. The cord 50 may be coated with a polymer, such as PVC. The cord 50 has a first end 501, and a second end 502 opposite the first one. The cord 50 can be inserted first end 501 first through the upper lacrimal punctum 12 or through the lower lacrimal punctum 15, along the upper 13 or lower 16 canaliculus, respectively, until reaching the nasal cavity. This technique can be used both to unblock the natural lacrimal duct (i.e. from the lacrimal sac to the nasal cavity via the natural conduit) or in the surgery whereby an artificial canal is made from the lacrimal sac to the nasal cavity (see figures 3A-3D). In other words, the cord is meant to be inserted along the natural lacrimal canal or duct, or an alternative thereto (created through surgery as described in the Background section, in this case to keep it from closing up before scarring). The first end 501 of the cord 50 is preferably blunt or rounded. The thickness of the cord 50 depends on the patient (from newborns up to adults and the elderly) and preferably ranges between 0.40 and 0.95 mm in diameter.

The cord 50 has at least two holes or housings ending in respective cul-de-sacs. In other words, these housings do not completely perforate or cut through the inner cross section of the cord 50, but rather form a notch surrounded by the rest of the material that makes up the cord. The purpose of these housings or holes is to press the rod 60 into them and thereby push the cord 50 so that it may be inserted more easily into the lacrimal duct. For this reason, the housings or holes must be large enough in size for the end of the rod to enter and fit inside of them, without leaving too much empty space around the rod, to keep it from slipping or moving out of the notch or housing. Therefore, the width or diameter of the housings must be similar to the cross section (or diameter) of the end of the rod that will be inserted, and they must be deep enough for the rod to fit in without actually passing through the cord. These housings preferably penetrate to a certain depth in the longitudinal direction of the cord, such that the piece of rod inserted therein may be longer than the thickness of the cord 50, due to the fact that the hole or housing runs substantially along the longitudinal axis of the cord 50, as may be see in figure 5B, for example. The length or depth of each housing in said direction substantially longitudinal to the axis of the cord varies approximately by between 3 and 10 mm. Figures 5A and 5B show three housings 51 52 53: a first housing 51 near to the first end 501 of the cord 50 (preferably at a distance that varies between 5 and 10 mm from the first end 501); a second housing 52 near to the second end 502 of the cord 50 (preferably at a distance of approximately 5 mm from the second end 502); and a third housing 53 situated in a portion of the cord 50 that is between the first and second housings 51 52. Preferably there are at least three housings 51 52 53. In this example, the housing 53 situated between the first and second housings 51 52 is closer to the second housing 52 than to the first housing 51. In the example, the housing 53 is situated at approximately 10 mm from the second housing 52. The housings are preferably aligned with one another, i.e. they are arranged along a common longitudinal axis of the cord 50. In a possible embodiment, the holes are coloured to make them more visible. The length of the cord preferably varies between 30 and 45 mm. One of the advantages of the device is that no prior measurements are necessary, as is the case in conventional devices, since the housings make it possible not to have to measure beforehand. Optionally the cord 50 may be cut, thus reducing its length to approximately 10-20 mm, for example for stenosis of the lacrimal punctum, stenosis of the lacrimal canaliculus, or sections of the lacrimal canaliculus.

As has been stated, the purpose of the housings is to press or insert the rod or mandrel 60 up to the cul-de-sac. The rod 60 is made of a substantially stiff biocompatible material. The rod 60 is preferably made of metal. Alternatively, the rod 60 may be made of a polymer (for example Teflon®). By way of example, and by no means limitation, the rod 60 is made of biocompatible stainless steel. The diameter of the rod 60 preferably varies between 0.3 and 0.8 mm. Therefore, the width or diameter of the cross section of the holes or housings in the cord 50 likewise varies between 0.3 and 0.8 mm (and, as has been stated, their length varies between approximately 3 and 10 mm). The rod 60 may comprise a handle 63 so that it is easier to manoeuvre. The handle may be made of any material that a person with average skill in the art deems appropriate. The handle may have any shape, but the preferred choice is a shape that does not make it difficult for the surgeon or medical staff to manoeuvre the rod. The end 61 of the rod 60 meant to be inserted or pressed into the housings of the cord 50 is preferably blunt or rounded.

The cord 50 may further comprise a plug or cap 57, preferably joined to the second end 502 of the cord 50, intended to be fixed or anchored in the lacrimal punctum. The plug or cap 57 is made of a biocompatible material, which may be the same material as the one used to manufacture the cord. This material is preferably a polymer, and more preferably silicone. Figure 6A shows an expanded view of the end 502 of the cord in figures 5A and 5B. The plug (cap) 57 is arranged on this end 502. The plug (cap) 57 is preferably continuous with the rest of the cord 50, i.e. the plug (cap) 57 may be considered the culmination of the cord 50 at the second end 502 of the cord. The plug (cap) is preferably perforated along its largest axis 573, so that tears can pass through. The plug (cap) is formed by a main hollow body, tube or neck 571 (inner tube 573), designed for one of its ends 572 to fit into the ampulla at the beginning of the lacrimal canaliculus and continue through the canaliculus. At the opposite end, the tube or neck 571 ends in a lip, edge or projection 575, preferably ring or collar shaped, meant to stay outside the patient and keep the assembly (plug or cap) from travelling into the lacrimal duct. The diameter of this lip or edge 575 is greater than the diameter of the main body or tube 571. This type of plug (cap), with a wider outer portion meant to stay outside the lacrimal orifice is called a punctal plug (cap). The plug (cap) 57 is joined laterally, preferably so that it is substantially perpendicular, to the longitudinal axis of the cord 50 by means of a portion of the main body, tube or neck 571. Preferably, the plug 5 (cap) 7 acts as the end 502 of the cord 50, closing it or ending it. In alternative embodiments, the plug (cap) could be joined to the cord forming an angle other than 90º.

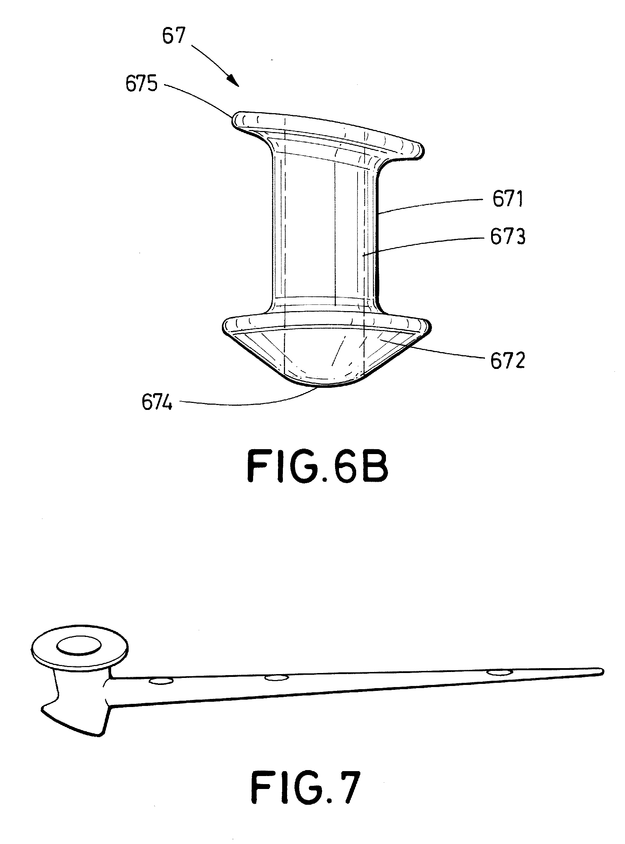

Figure 6B shows an alternative embodiment of the plug (cap) 67. Although this figure illustrates a loose plug (cap), separated from the cord, this separation is merely to better illustrate the plug, as the plug (cap) 67 is preferably joined to the cord 50 in a way similar to the previous description of how the plug 57 is joined. The plug (cap) 67 is also perforated along its largest axis, forming a hollow inner tube 673, so that tears can pass through. The plug (cap) 67 is formed by a main hollow body, tube or neck 671 (inner tube 673), designed for one of its ends 672 to fit into the ampulla at the beginning of the lacrimal canaliculus and continue through the canaliculus. In this case, unlike plug (cap) 57, plug (cap) 67 has a lip at this end 672 with a conical or frustoconical shape (i.e. in the shape of a cone or a frustum) or a sort of blunt or rounded arrowhead 674. This arrowhead 674 is meant to fit into the beginning of the lacrimal canaliculus, closing off the duct. At the opposite end, the main tube or neck 671 ends, as with the plug (cap) 57, in a lip, edge or projection 675, preferably ring or collar shaped, meant to stay outside the patient and keep the assembly (plug or cap) from travelling into the lacrimal duct. The diameter of this lip or edge 675 is greater than the diameter of the main body or tube 671. For example, the main body 671 may have an outer cross section (outer diameter) that varies between 0.4 and 1 mm (preferably approximately 0.8 mm); the widest part of the lip 672 may have a diameter that varies between 1 and 2 mm (preferably approximately 1.4 mm); and the lip or edge 675 of the opposite end may have a diameter that varies between 1 and 4 mm (preferably approximately 1.5 mm). This lip or edge 675 may be asymmetrical with respect to the main body 671 and be arranged such that it is not perpendicular to said body 671, as shown in figure 6B. The total length of the plug (cap) from end to end is approximately 2 mm. The dimensions vary based on the age of the person who is going to use the stent (note that this device is intended for both newborns and adults). If it is shaped like an arrowhead, the latter is designed to have a blunt or rounded tip so as not to harm the lacrimal punctum. As in the previous case, the rounded arrowhead is designed to fit into the lacrimal punctum, closing it off, and the diameter of its chosen cross section is to be similar to the frustoconical version. In other words, the end of the plug (cap) 67 with the smaller cross section substantially has the shape of a truncated or rounded arrowhead that fixes to the inside of the lacrimal punctum.

As has been mentioned, the inside of the plug (cap) 57 67 is perforated. This means that the plug (cap) forms a hollow tube that allows tears to flow inside of it. The hollow inner tube along the longitudinal axis of the plug 57 67 allows tears to flow from the outside into the canaliculus, which, although it is occupied by the cord 50, the cord 50 allows tears to flow toward the nasal cavity.

As has been mentioned, the cord 50 is preferably joined to the plug (cap) 57 67 perpendicularly, and more preferably the notches or holes to house the mandrel or rod are located on this same side of the cord (i.e. lined up along the cord, on the same side, there is first the plug and then one notch or hole after another).

In other words, the assembly is used as follows: the first end 501 of the cord 50 is placed at the entrance of the upper lacrimal punctum 12, and the end 61 of the rod 60 is inserted into the first housing 51 of the cord 50, until it is pressed into the cul-de-sac of the corresponding housing. One then begins to push the cord 50 with the rod 60, guiding it with the rod 60 along the upper canaliculus 13. Alternatively, the cord may be inserted in a similar manner through the lower lacrimal punctum and its corresponding canaliculus. When the next housing (53 in figure 5) - which may be closer to or farther from the first housing 51 - is at the level of the lacrimal punctum that the cord is being inserted through (upper or lower), the rod 60 is taken out of the first housing and pressed or inserted into the next housing, thereafter continuing to push or guide the cord 50. This process is repeated until the first end 501 of the cord 50 has reached its destination, which is the nasal cavity, either through the natural duct or through the surgical duct.

This process may be carried out both when the aim is to unblock the natural lacrimal canal, and when using an artificial canal made through surgery (for example through laser surgery, inserting a laser probe through one of the puncta lacrimalia and its respective canaliculus until it is inserted into the lacrimal sac, perforating it along with the nasal wall, and making the laser probe emerge into the nasal cavity). The tears run through the canaliculus outside the cord. Nevertheless, this is a temporary intubation (maximum six months). Figure 7 shows a diagram of an assembly formed by cord and plug (cap), in accordance with another possible embodiment of the invention.

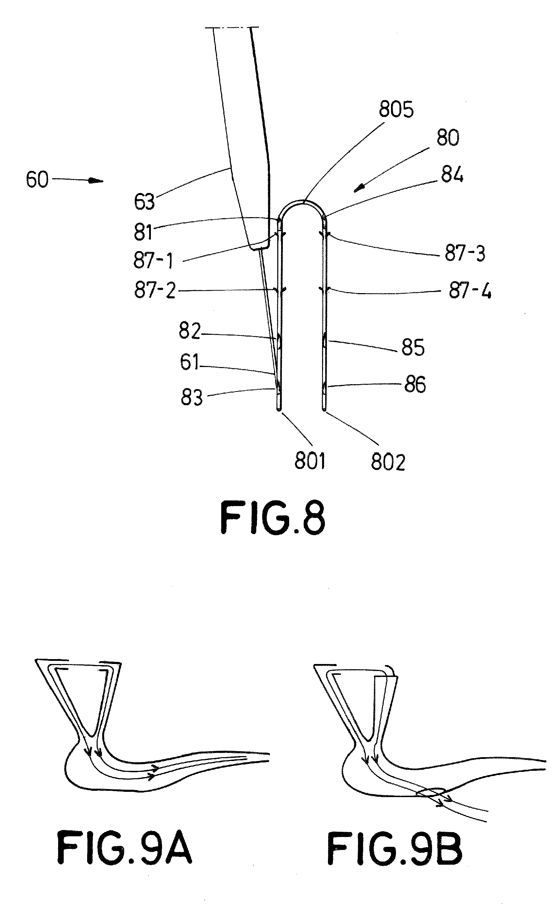

Figure 8 shows another possible embodiment of the invention that is similar to the previous one, but based on a bicanalicular or bicanalicular-nasal cord, as it is designed to be inserted by one of its ends 801 into one of the puncta lacrimalia and its corresponding canaliculus, and by its other end 802 into the other lacrimal punctum and its corresponding canaliculus. As in the previous case, the cord 80 can be used both to unblock the natural duct or the artificial canal made through surgery. The cord 80 is made of the same material and has the same thickness as the cord 50 of the previous embodiment. As with the previous version, the cord 80 has a plurality of holes 81 82 83 84 85 86 for pressing or inserting a rod or mandrel 60, which also has the same characteristics as the one described in the previous embodiment. Since the bicanalicular cord 80 will be inserted through two ducts instead of one, it must be approximately twice as long as the monocanalicular cord 50, and must have at least two push holes per duct (81 82 83; 84 85 86), i.e. at least four push holes. To be inserted, the cord 80 is folded in half at a midpoint 805, in order to insert each end 801 802 through each one of the canaliculi (upper and lower). As has been mentioned, each one of these halves has a series of openings or holes or housings (in figure 8, 81 82 83 on one half and 84 85 86 on the other half) and corresponding cul-de-sacs, which are similar in shape and size to those of the cord 50 in figure 5A, in order to press a rod therein and thus push the cord. Each half preferably has at least three holes or openings or housings, arranged at different distances from the midpoint. Preferably, these distances are similar to those described in relation to the cord 50 in figure 5A, but bearing in mind that in this case the cord is meant to be inserted through both tear ducts and folds approximately in the middle. By way of example, a first hole 81 84 is situated at approximately 15 mm from the midpoint, another hole 82 85 at approximately 25 mm from the middle, and a third hole 83 86 at approximately 5-10 mm away from each tip or end 801 802 of the cord 80.

The cord 80 in figure 8 may be inserted from a lacrimal punctum along the corresponding canaliculus, by both of its ends, i.e. at end 801 the cord 80 is inserted and pushed by the rod 60 along the upper canaliculus, for example, whilst at end 802, the one opposed the previous end 801, the cord 80 is inserted and pushed by the rod 60 along the lower canaliculus, for example. As has been indicated, because it is meant for double insertion through both of an eye's canaliculi, this cord 80 is longer than the previous one. Its total length preferably measures between 90 and 105 mm. The distance between the hole closest to each end and said end 801 802 is preferably the same as in the previous case. Moreover, the separation between holes is also preferably the same as in the previous case.

Each one of these halves has one or more pairs of tabs or anchors 87-1 87-2 87-3 87-4 folding out from the cord (silicone tabs if the cord is made of silicone), forming a sort of arrow, to stabilize the cord 80 when it enters the sac. This makes it possible to forgo nasal retrieval. For example, at least one pair of tabs or anchors 87-1 87-3 is situated near the hole 81 84 and cul-de-sac situated approximately 15 mm from the midpoint 805. It is also possible to add a second pair of tabs or anchors 87-2 87-4 near the hole 82 83 situated approximately 25 mm from the middle (in each piece of cord 80).

With respect to the cord 50, as shown in figure 5A, the monocanalicular cord 50 preferably does not have anchors or anchorings, but rather the plug itself serves to anchor it.

This stent may be used in bicanalicular intubation of the entire natural lacrimal duct (often in children) or in an artificial duct created through surgery.

When the bicanalicular intubation is carried out in the natural lacrimal duct (often in children), the closest anchor or arrow to each end of the cord 80 becomes fixed in the proximal part of the sac where it joins the common canaliculus, and the second anchor or arrow becomes fixed in the middle of the sac. One of its greatest advantages is that it does not require nasal retrieval. When the bicanalicular intubation is carried out in the artificial lacrimal duct obtained through laser surgery (creating a fistula between the sac and nose with a laser) (often in adults), the closest anchor to the middle of the cord 80 also becomes fixed in the proximal portion of the sac, but the second anchor passes through the fistula carried out via laser between the sac and the nose, and becomes fixed in the nose. Nasal retrieval is not necessary in this case either. Figures 9A and 9B show a diagram of the anchors when they are in the lacrimal duct, both natural and surgical.

Another versatile aspect is that the stent can be cut after approximately 15 mm, leaving a stent to intubate just the canaliculi, as in self-supporting stents.

In other words, the assembly illustrated in figure 8 is used as follows: the first end 801 of the cord 80 is placed at the entrance of the upper lacrimal punctum 12, and the end 61 of the rod 60 is inserted into the first housing 83 of the cord 80, until it is pressed into the corresponding cul-de-sac. One then begins to push the cord 80 with the rod 60, guiding it with the rod 60 along the upper canaliculus 13. When the next housing (82 in figure 8) - which may be closer to or farther from the first housing 83 - is at the level of the lacrimal punctum that the cord is being inserted through (upper or lower), the rod 60 is taken out of the first housing and pressed or inserted into the next housing, thereafter continuing to push or guide the cord 80. This process is repeated until the first end 801 of the cord 80 has reached its destination, which is the nasal cavity, either through the natural duct or through the surgical duct. Lastly, the same operation is repeated through the other canaliculus with the other end 802 of the stent, such that the stent becomes stabilized through the anchors 87-1 87-2 87-3 87-4.

This process may be carried out both when the aim is to unblock the natural lacrimal canal, and when using an artificial canal made through surgery (for example through laser surgery, inserting a laser probe through one of the puncta lacrimalia and its respective canaliculus until it is inserted into the lacrimal sac, perforating it along with the nasal wall, and making the laser probe emerge into the nasal cavity). The tears run through the canaliculus outside the cord 80. Nevertheless, this is a temporary intubation (maximum six months).

Once the cord has been inserted (whether it is the double cord 80 or the single cord 50), it remains inside the canal for between two and six months, acting as a stent, keeping the canal from closing up or becoming obstructed. To take it out, the cord 50 80 is removed through the eye (pulling it through the lacrimal punctum 12 15 by its end), when it has been determined that the lacrimal canal (whether artificial or natural) has healed and is not at risk of closing back up.

As can be seen, the assembly of the invention makes it possible to insert the cord along the recently created canal or duct, or the natural duct. This prevents injury to the nasal cavity or to the newly created lacrimal canal itself, as the case may be. Moreover, since no manoeuvring needs to be done through the nostrils, the process is simple and does not require an otorhinolaryngologist to be present. Moreover, being able to use a single cord for both ducts or canaliculi simplifies and economizes the necessary materials (a single cord and a rod for pushing) and eases the patient's recovery.

In short, the proposed monocanalicular device has the advantage, over conventional monocanalicular devices, that the mandrel or rod does not go into the hollow part (longitudinal axis) of the silicone stent, but rather outside, as it is inserted in the various notches in the cord. In this way, if one has already reached the floor of the nasal cavity and there is still a large amount of cord to be inserted, the mandrel or rod may then be removed and inserted into another notch that is farther away from the end, in order to continue pushing the stent through the lacrimal duct.

As for the bicanalicular version, the proposed device has the advantage, over conventional bicanalicular devices, that the mandrel or rod does not go into the hollow part (longitudinal axis) of the silicone stent, but rather outside, as it is inserted in the various notches in the cord. This makes it easer to place in the lacrimal duct. As opposed to conventional devices, it also prevents having to retrieve the stent through the nose, thus avoiding traumatic injury and difficult handling. In short, the bicanalicular stent need not be tied off, as is the case with conventional devices.

Thus, in both cases the mandrel or rod used is independent of the tube (the cord in this case), i.e. the rod does not follow after the tube or cord.

Once the stent has been placed, there needs to be a way to keep it in the correct position, since it will not be retrieved through the nose nor tied off. To do this, in the monocanalicular case a plug is used, and in the bicanalicular case the aforementioned anchors are used.

Also, the invention is not limited to the specific embodiments described herein, but rather encompasses the variations that one skilled in the art could make (e.g. in terms of choice of materials, dimensions, components, design, etc.), within the scope of what may be deduced from the claims.

## Claims

1. A device for treating obstruction of the lacrimal ducts, comprising a cord (50, 80) made of biocompatible material, the cord (50, 80) having a first end (501, 801) and a second end (502, 802) opposite the first one, said first end (501, 801) being blunt or rounded, the device being **characterised in that** the cord (50, 80) is designed to be inserted first end (501, 801) first through an eye's lacrimal punctum (12, 15) along a respective canaliculus (13, 16) until reaching the nasal cavity, said cord (50, 80) comprising a plurality of housings (51, 52, 53; 81, 82, 83) ending in respective cul-de-sacs, designed to receive an end of a rod (60) such that, when an end of the rod (60) is inserted into a housing (51, 52, 53; 81, 82, 83) and said end of the rod is pressed into the housing, the cord (50, 80) is pushed by the rod (60) from the outer part of the cord (50, 80) into the lacrimal duct.

2. The device of claim 1, wherein the housings are aligned with one another, i.e. they are arranged along a common longitudinal axis of the cord (50, 80).

3. The device of any of the preceding claims, wherein the width or diameter of each housing varies between 0.3 and 0.8 mm, and its length or depth varies between 3 and 10 mm.

4. The device of any of the preceding claims, wherein said cord (50, 80) is made of silicone.

5. The device of any of the preceding claims, wherein said cord (50, 80) has a PVC coating.

6. The device of any of the preceding claims, which further comprises a plug or cap (57, 67, 77) designed to plug the lacrimal punctum through which the cord (50) has been inserted, after the latter has been inserted into the lacrimal duct.

7. The device of claim 6, wherein said plug or cap (57, 67) is joined to the second end (502) of the cord (50).

8. The device of any of the claims 1 to 5, wherein the second end (802) of said cord (80) is designed to be inserted through an eye's second lacrimal punctum (12, 15) along the respective canaliculus (13, 16) until reaching the nasal cavity, where the length of cord (80) that follows said second end (802) comprises a plurality of housings (84, 85, 86) ending in respective cul-de-sacs, designed to receive an end of said rod (60) such that, when said end of the rod is pressed into a housing, the length of cord (80) is pushed into the lacrimal duct.

9. The device of claim 8, wherein in the vicinity of at least one of the housings (81, 82, 83, 84, 85, 86) of said cord (80), the cord (80) further comprises at least one anchor (87-1 87-2 87-3 87-4) in the form of a projection to help fix the cord (80) in the lacrimal duct once it has been inserted therein.

10. The device of any of the preceding claims, wherein said cord (50, 80) is designed to be introduced along a natural lacrimal canal or duct or a lacrimal canal or duct made through surgery.

11. A monocanalicular or bicanalicular intubation assembly, comprising:
- a device according to any of the claims 1 to 10, and
- a substantially stiff metal or Teflon® rod (60), comprising a blunt or rounded end designed to be inserted into one of the housings (51, 52, 53; 81, 82, 83, 84, 85, 86) of said cord (50, 80) in order to move the cord forward along the lacrimal duct.

12. The assembly of claim 11, wherein the diameter of said rod varies between 0.3 and 0.8 mm.

13. A use of the device or assembly of the preceding claims, in a surgical treatment.

14. The device according to the claims 1 to 10 or the assembly according to any of the claims 11 or 12, for use thereof in a surgical method that comprises creating an artificial lacrimal duct or using a natural duct between a patient's lacrimal sac (14) and nasal cavity (18).
